# EUROPEAN PATENT SPECIFICATION

(11) **EP 1 066 850 B1**
(45) Date of publication and mention of the grant of the patent: **09.08.2006**
(21) Application number: 00109248.5
(22) Date of filing: 28.04.2000
(51) Int. Cl.: A61M 15/00

(54) **Medical Nebulizer**
Medizinischer Vernebler
Nébuliseur médical

(30) Priority: 08.07.1999 SE 9902627
(43) Date of publication of application: 10.01.2001
(73) Proprietor: Maquet Critical Care AB, 171 95 Solna (SE)
(72) Inventor: Sjöholm, Gösta, 168 69 Bromma (SE)
(74) Representative: Stein, Jan Anders Lennart

(56) References cited:
- EP-A- 0 111 163
- EP-A- 0 642 802
- WO-A-96/28205
- GB-A- 2 272 389
- US-A- 3 812 854
- US-A- 5 443 059

## Description

The present invention relates to a medical nebulizer and in particular to a medical nebulizer for providing a nebulized physiologically active liquid into an inspiration gas flow of a mechanical breathing aid.

Medical nebulizers are often used to deliver a metered dose of a physiologically active liquid into an inspiration gas stream for inhalation by a recipient. They generally operate to generate liquid droplets which form an aerosol with the inspiration gas. In other circumstances medical nebulizers may be used to inject water droplets into an inspiration gas stream to provide gas with a suitable moisture content to a recipient, this is particularly useful where the inspiration gas stream is provided by a mechanical breathing aid such as a respirator, ventilator or anaesthetic delivery system.

The term "medical liquid" will be used to describe any liquid, whether or not it is physiologically active, which is to be supplied to the airways of a recipient.

One known medical nebulizer is described in WO 95/01137 and is a hand held device which operates to eject droplets of a medical liquid into a passing air stream (inspiration gas stream) which is generated by a recipient's inhalation through a mouth piece. This known device comprises a reservoir for the medical liquid which is connected to a capillary nozzle via a pump which operates directly on liquid within the capillary to eject liquid droplets through the nozzle and into an inspiration gas flow conduit. The pump means comprises a bubble jet or piezoelectric pump, both of which are pulsed to eject a droplet through the nozzle with each pulse. Control means is also provided to regulate the dose based on the number of pulses provided to the liquid by the pump, for example by regulating the time that a drive signal of known frequency is applied to the piezoelectric element of the pump.

One problem with this device is that dosage is dependent on the pulse frequency. This limits the device to the delivery of relatively small doses unless plurality of nozzle and pump arrangements are employed. This would increase the overall size of the device. Moreover when the piezoelectric pump is used the supply of a pulse to the piezoelectric crystal can stimulate the crystal to produce a pulse train of typically 5 to 6 pulses and lead to an inaccurate dose being provided. Furthermore, the pump is necessarily relatively small as it must act only on liquid within the capillary. The pumping power of such a pump tends to be limited so that at small capillary bore sizes flow resistance can become a problem, limiting the minimum droplet size to typically microlitres.

It is an aim of the present invention to provide a medical nebulizer which overcomes the pumping problems associated with the known device.

Accordingly there is provided a medical nebulizer described in and characterised by the present Claim 1. By arranging for the pump to supply a continuous stream of liquid to be nebulized with the aid of a stimulator for vibrating the nozzle a metered dose of medical liquid can be provided in droplet form without the need to monitor the number of droplets.

Moreover, by using a pump which does not act directly on liquid within the capillary a more powerful and less expensive pump, for example a syringe pump, may be used. This enables the capillary bore size to be reduced over known nebulizer and picolitre sized droplets may be supplied. The finer droplet formation allows a more homogeneous vapour to be formed in the inspiration gas stream and a more efficient uptake of the medical agent by the patient.

Usefully the reservoir and the nozzle may be releasably couplable. In this way the reservoir may be readily disposed of, replaced or removed for sterilizing as necessary. This also provides an administrative benefit in that an estimate of the amount delivered can be readily obtained from a count of the used reservoir cartridges, enabling for example, stocks to be timely replenished.

Usefully, the pump may be operated continuously and suitable valving means provided, such as micro-valves which have particularly well defined cut on and off characteristics, to limit the supply of liquid in order to deliver the predetermined dose from the nebulizer. In this manner inaccuracies in the delivered dose due to the start up and shut down characteristics of the pump may be alleviated.

In order to co-ordinate the delivery of the liquid dose with an inspiration of a recipient, which may be either before, during or after an inspiration, a flow meter may be provided in the flow path of the inspiration gas and adapted to control the delivery dependent on a sensed gas flow.

Embodiments of the present invention will now be described by way of non-limiting examples only and with reference to the drawings of the accompanying figures of which:
Fig. 1 shows a schematic block diagram of a ventilator apparatus having a nebulizer according to the present example.
Fig. 2 shows a schematic illustration of a nebulizer according to the present invention usable in the ventilator arrangement of Fig. 1.
Fig. 3 shows a schematic illustration of a "hand-held" nebulizer according to the present invention.
Fig. 4 shows a schematic illustration of a further embodiment of the nebulizer according to the present invention.

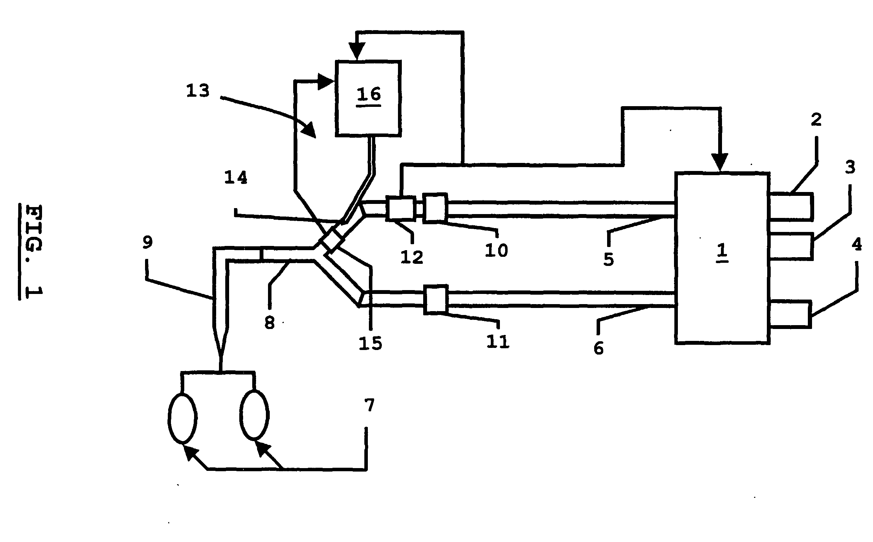

Considering now Fig. 1, a ventilator apparatus is generally shown in which electronic control signal flow paths are shown by the arrows connecting elements of the apparatus. A conventional ventilator 1 is shown having a number (here two) of input gas lines 2,3 for different gasses and an evacuation gas line 4. Patient breathing conduits 5,6 are provided for transporting breathing gas between the ventilator 1 and lungs 7 of a patient via a Y-piece 8 and endo-tracheal tube 9. The common branch of the Y-piece 8 is connected to the tube 9 and the separate branches are connected to respective conduits 5,6 in order to provide a flow path 5,8,9 for breathing gas from the ventilator 1 to the patient (inspiration gas flow) which is separate from a flow path 9,8,6 for breathing gas from the patient to the ventilator 1 (expiration gas flow). One-way valves 10,11 are placed in associated conduits 5,6 in order to ensure the correct direction of travel of breathing gas. A flow meter 12 is placed in the inspiration flow path 5,8,9, for example downstream of the one-way valve 10 in the inspiration gas flow direction.

A nebulizer 13 according to the present invention is also provided for supplying water vapour to the inspiration gas flowing to the patients lungs 7 through a capillary nozzle 14 connected to the flow path 5,8,9. The nozzle 14 is disposed to provide the liquid preferably as close to the patient as possible and typically at the Y-piece 8.

A moisture meter 15 is also provided to monitor the moisture content of the inspiration gas after passing the nozzle 14 and to provide an indication of the measured moisture content to a control/dispenser unit 16 of the nebulizer 13 where it is used to control the dosage in combination with gas flow information from the flow meter 12.

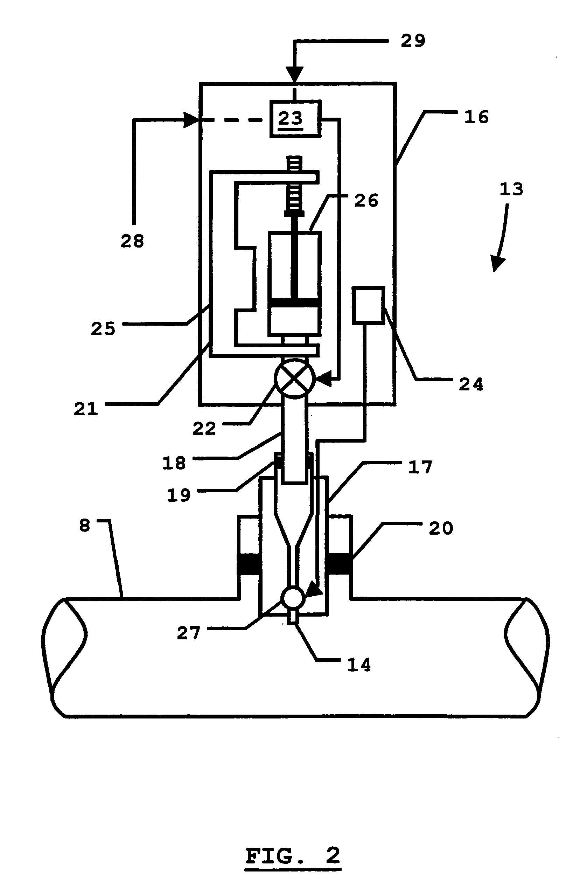

The operation of the nebulizer 13 will now be further explained with reference also to Fig. 2. The nebulizer 13 shown in Fig. 2 comprises the control/dispenser unit 16 and a nozzle unit 17 which are in fluid communication by means of a conduit 18 which, at one end, is releasably connected to the proximal end of the capillary nozzle 14. This releasable connection may be made using any conventional fluid tight connecting means and is here a push fit connection through an O-ring seal 19. The nozzle unit 17 is also releasably connected to the Y-piece 8 of the ventilator apparatus of Fig. 1, again using a push fit O-ring seal 20. It will be apparent to those skilled in the art that with this arrangement various parts of the nebulizer 13 may be conveniently replaced or removed for cleaning.

The opposite end of the conduit 18 is connected to a syringe pump 21 via a controllable on/off valve 22 which are within the control/dispenser unit 16. Also within this unit 16 is disposed a microprocessor based control unit 23, an oscillator driver unit 24 and a pump power supply 25.

The pump power supply 25 is adapted to continuously pressurise liquid within the syringe reservoir 26 of the syringe pump 21 during the operation of the nebulizer 13 with the dosage being controlled via the on/off valve 22 and the control unit 23.

The oscillator driver unit 24 is also adapted to continuously supply a high frequency (typically in the region of 1 MHz) drive signal to a piezoelectric oscillator 27 which is disposed within the nozzle unit 17 to vibrate the distal end of the capillary nozzle 14 to stimulate droplet formation in a manner similar to that well known in the art of ink-jet printing. The oscillator unit 24 may be adapted to vary the frequency of the drive signal in dependence of the liquid used, its flow rate and preferred droplet size in order to enhance the operational flexibility of the nebulizer 13 (it is well understood that droplet size depends on, amongst other things, the liquid flow rate through the nozzle 14, the viscosity of the liquid and vibrational frequency).

In use power is provided to the oscillator drive unit 24 and the pump power supply unit 25 from a mains supply via an on/off switch (not shown) accessible externally of the control/dispenser unit 16 and which operate continuously whilst the nebulizer 13 is switched on. An input signal 28 from the moisture meter 15 of Fig. 1, indicative of the moisture content of inspiration gas, and an input signal 29 from the flow meter 12 of Fig. 1, indicative of the presence and magnitude of an inspiration gas flow, are provided to the control unit 23. The dosage of water from the reservoir 26 necessary to provide the desired relative humidity (which may be user variable or a fixed value) of the inspiration gas is then determined within the control unit 23 and a signal 30 is output to operate the valve 22 to achieve the determined dose. In the present case it is assumed that the flow of water from the syringe pump 21 is predetermined and programmed into the control unit 23. However a flow meter (not shown) may be included to directly measure the flow of fluid within the conduit 18, the value of which will be used within the control unit 23 in the regulation of the flow through the valve 22 in order to achieve the determined dose.

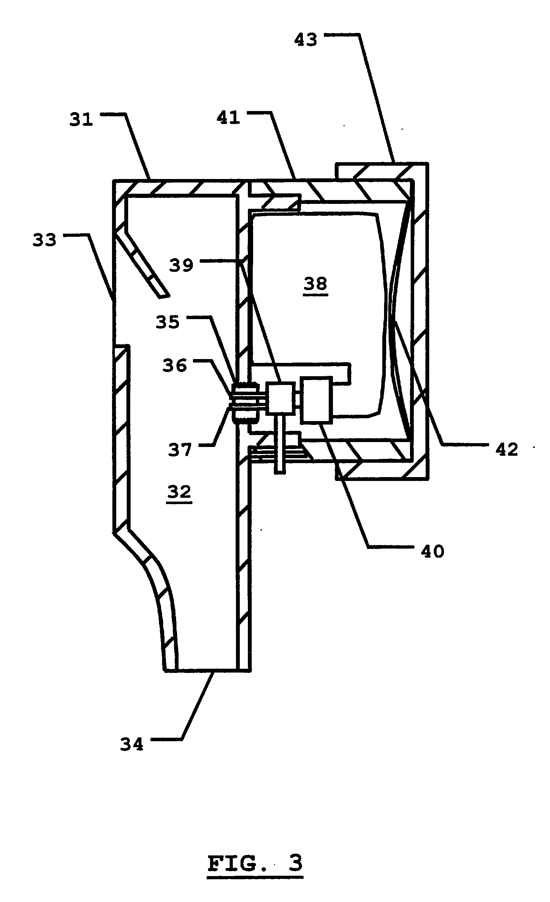

Turning now to Fig 3. a nebulizer is shown which is intended for use without a mechanical breathing aid. A mouthpiece 31 defines an inspiration gas conduit 32 having an inlet 33 open to the atmosphere and an outlet 34 intended for insertion into a patient's mouth. A nozzle section 35 is releasably screwed into the mouthpiece 31 so that liquid droplets can pass from distal ends of a number of capillary nozzles (here two) 36,37 into the inspiration gas conduit 32. A piezoelectric oscillator 39, is mechanically coupled to the nozzles 36,37 to vibrate their distal ends and is provided with an externally accessible electrical contact 40 to which oscillator drive signals can be applied. It will be appreciated that the nebulizer of Fig. 3 may be readily modified to include an oscillator drive unit, with or without a battery power supply, to provide a more fully portable nebulizer.

Proximal ends of the nozzles 36,37 are connected to a common liquid reservoir 38 in which is contained a physiologically active liquid. The liquid reservoir 38 is releasably connected to a one-way valve 40, for example using a push fit or screw thread connection for ease of replacement. The valve 40 is arranged to prevent through flow towards the gas conduit 32 until pressure from the liquid exceeds a pre-set amount just above atmospheric. This amount is chosen so that liquid from reservoir 38 cannot flow through the valve 40 under the pressure of its own weight.

The reservoir 38 comprises a readily deformable container which is housed in a holder 41 having at least one wall section 42 formed of elastically deformable material such that as the section 42 is squeezed the reservoir deforms to expel liquid through the valve 40. A rigid end-cap 43 is provided to push fit over the holder 41. This cap 43 provides physical protection for the elastic wall section 42 against accidental deformation and unintended release of liquid from the reservoir 38. The reservoir 38 may be sized to control the dose provided to the patient so that a single dose will be delivered when the reservoir 38 is fully squeezed.

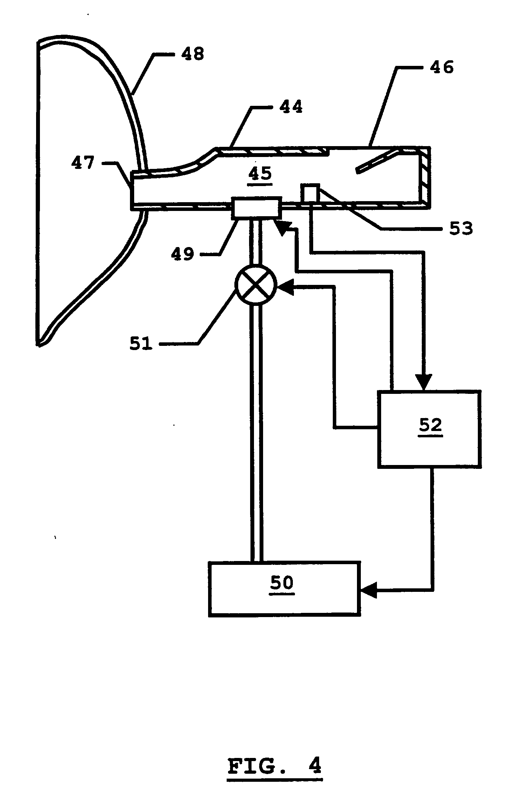

Fig. 4 shows a nebulizer useful, for example, in the treatment of weak or unconscious patients who are breathing without the aid of a mechanical breathing aid. A common flow section 44 defines a gas conduit 45 having a first aperture 46 open to the atmosphere and a second aperture 47 intended for gas connection to a patient's mouth via a face mask 48. A nebulizer unit 49 is connected to the gas conduit 45 through a wall of the common flow section 44 so that liquid droplets can pass from distal ends of a number of capillary nozzles, which may be vibrated severally or separately using a piezoelectric vibrator within the nebulizer unit 49. The number of nozzles actually used to supply the liquid droplets can be less than the total number of nozzles provided and selected, for example, dependent on a desired rate of supply of nebulized liquid. Moreover, nozzles having a variety of different sizes may be included to further increase the flexibility in supply characteristics of the nebulizer.

A pump unit 50, such as the syringe pump described with respect to Fig 1., is operably connected to the capillary nozzles of the nebulizer unit 49 via a valve unit 51 and includes a medical liquid reservoir (not shown) from which liquid is pumped through the nozzles of the unit 49 to form a vapour in gas within the conduit 45. A control unit 52, such as a dedicated microprocessor or a suitably programmed personal computer, is also provided to control the operation of the vibrator within the nebulizer unit 49, the valve unit 51 and the pump unit 50 to supply the fluid in dependence of a signal from a flow sensor 53 within the gas conduit 45 of the common flow section 44 indicating a patients inspiration phase. The control unit 52 may be programmed to operate the pump unit 50 to tend to supply a continuous stream of liquid through the nozzle section 49 and to control the valve unit 51 to open and close and provide a desired amount of medical liquid to the patient. Alternatively, the pump unit 50 may be operated intermittently in order to provide the desired amount of medical liquid. In both alternatives this desired amount may be either pre-set in the control unit 52 provided as a user input where the control unit 52 is also provided with a user interface.

## Claims

1. A medical nebulizer comprising
a reservoir (26; 38) for medical liquid;
a capillary nozzle (14; 36; 37) having a proximal end for receiving liquid from the reservoir and a distal end connectable to an inspiration gas flow path (5, 8,9; 32; 45) to deliver liquid droplets into an inspiration gas flow;
pump means (21; 42; 50) for supplying liquid from the reservoir through the capillary nozzle;
means (22, 23; 51; 52) for regulating the delivery of the liquid to a predetermined amount; and
a stimulator (27; 39) for vibrating the distal end of the capillary nozzle to stimulate droplet formation;
**characterised in that**
the pump means is adapted not to act directly on the liquid within the capillary and to supply the liquid in a continuous flow throughout the delivery of the predetermined amount.

2. A medical nebulizer as claimed in claim 1, **characterised in that**
the means for regulating the delivery comprises valving means (22; 51) operable to limit the supply of liquid through the capillary nozzle to the predetermined amount.

3. A medical nebulizer as claimed in claim 1, **characterised in that**
the means for regulating the delivery (52) is adapted to regulate the power supply to the pump (50) to limit the supply of liquid through the nozzle to the predetermined amount.

4. A medical nebulizer as claimed in any of claims 1-3, **characterised in that**
a flow meter (53) is positionable in the flow path (45) of the inspiration gas and that the means for regulating the delivery (52) is operable to regulate the delivery of liquid dependent on a detection of an inspiration flow by the flow meter.

5. A medical nebulizer as claimed in any preceding claim **characterised in that**
the pump means comprises a syringe pump (21).

6. A medical nebulizer as claimed in claim 5, **characterised in that**
the syringe pump is provided with a reservoir (26) releasably couplable to the proximal end of the capillary nozzle.

7. A medical nebulizer as claimed in any preceding claim **characterised in that**
the capillary nozzle is connectable to an inspiration gas flow path (5, 8, 9) of a mechanical breathing aid (1).

8. A medical nebulizer as claimed in any preceding claim, **characterised in that**
there is provided a number of further nozzles (36; 37), individually or collectively vibratable to stimulate droplet formation.

## Patentansprüche

1. Medizinischer Vernebler, umfassend
einen Speicher (26; 38) für medizinische Flüssigkeit,
eine Kapillardüse (14; 36; 37) mit einem proximalen Ende zur Aufnahme von Flüssigkeit aus dem Speicher und einem distalen Ende, das an einen Inspirationsgasströmungsweg (5, 8, 9; 32; 45) angeschlossen werden kann, um Flüssigkeitstropfen in einen Inspirationsgasfluss abzugeben,
Pumpenmittel (21; 42; 50) zum Fördern von Flüssigkeit aus dem Speicher durch die Kapillardüse,
Mittel (22, 23; 51; 52) zum Einstellen der Abgabe der Flüssigkeit auf eine vorgegebene Menge, und
einen Stimulator (27; 39), um das distale Ende der Kapillardüse in Schwingung zu versetzen und so die Tropfenbildung anzuregen,
**dadurch gekennzeichnet, dass**
die Pumpenmittel so angepasst sind, dass sie nicht direkt auf die Flüssigkeit in der Kapillare wirken und die Flüssigkeit während der gesamten Abgabe der vorgegebenen Menge in einem kontinuierlichen Fluss zuführen.

2. Medizinischer Vernebler nach Anspruch 1, **dadurch gekennzeichnet, dass**
die Mittel zum Einstellen der Abgabe Ventilmittel (22; 51) umfassen, die betrieben werden können, um die Förderung der Flüssigkeit durch die Kapillardüse auf die vorgegebene Menge zu begrenzen.

3. Medizinischer Vernebler nach Anspruch 1, **dadurch gekennzeichnet, dass**
die Mittel (52) zum Einstellen der Abgabe so angepasst sind, dass sie die Energieversorgung zur Pumpe (50) einstellen, um die Flüssigkeitsabgabe durch die Düse auf die vorgegebene Menge zu begrenzen.

4. Medizinischer Vernebler nach einem der Ansprüche 1 bis 3, **dadurch gekennzeichnet, dass**
ein Durchflussmesser (53) in dem Strömungsweg (45) des Inspirationsgases positioniert werden kann und die Mittel (52) zum Einstellen der Abgabe betrieben werden können, um die Flüssigkeitsabgabe in Abhängigkeit von einem Erfassen eines Inspirationsflusses durch den Durchflussmesser einzustellen.

5. Medizinischer Vernebler, nach einem der vorhergehenden Ansprüche, **dadurch gekennzeichnet, dass**
die Pumpenmittel eine Spritzenpumpe (21) umfassen.

6. Medizinischer Vernebler nach Anspruch 5, **dadurch gekennzeichnet, dass**
die Spritzenpumpe mit einem Speicher (26) versehen ist, der lösbar mit dem proximalen Ende der Kapillardüse verbunden werden kann.

7. Medizinischer Vernebler nach einem der vorhergehenden Ansprüche, **dadurch gekennzeichnet, dass**
die Kapillardüse an einen Inspirationsgasströmungsweg (5, 8, 9) einer mechanischen Beatmungshilfe (1) angeschlossen werden kann.

8. Medizinischer Vernebler nach einem der vorhergehenden Ansprüche, **dadurch gekennzeichnet, dass**
eine Vielzahl weiterer Düsen (36; 37) vorgesehen ist, die einzeln oder gemeinsam in Schwingung versetzt werden können, um die Tropfenbildung anzuregen.

## Revendications

1. Nébuliseur médical comprenant
un réservoir (26, 38) à liquide médical ;
une buse (14, 36, 37) capillaire ayant une extrémité proximale pour recevoir du liquide du réservoir et une extrémité distale pouvant communiquer avec un trajet (5, 8, 9, 32 45) de courant de gaz d'inspiration afin d'envoyer des gouttelettes de liquide dans un courant de gaz d'inspiration ;
des moyens (21, 42, 50) de pompage pour envoyer du liquide du réservoir dans la buse capillaire ;
des moyens (22, 23, 51, 52) de régulation de l'administration du liquide en une quantité déterminée à l'avance ; et
un stimulateur (27, 39) pour faire vibrer l'extrémité distale de la buse capillaire afin de stimuler une formation de gouttelettes ;
**caractérisé en ce que**
les moyens de pompage sont conçus pour ne pas agir directement sur le liquide au sein du capillaire et pour envoyer le liquide en un courant continu pendant toute l'administration de la quantité déterminée à l'avance.

2. Nébuliseur médical tel que revendiqué à la revendication 1,
**caractérisé en ce que**
les moyens de régulation de l'administration comprennent des moyens (22, 51) à vanne pouvant fonctionner pour limiter l'envoi de liquide dans la buse capillaire à la quantité déterminée à l'avance.

3. Nébuliseur médical tel que revendiqué à la revendication 1,
**caractérisé en ce que** les moyens de régulation de l'administration (52) sont conçus pour réguler la puissance fournie à la pompe (50) pour limiter l'envoi de liquide dans la buse à la quantité déterminée à l'avance.

4. Nébuliseur médical suivant l'une quelconque des revendications 1 à 3,
**caractérisé en ce qu'**un débitmètre (53) peut être mis dans le trajet (45) de courant du gaz d'inspiration et **en ce que** les moyens de régulation de l'administration (52) peuvent fonctionner pour réguler l'administration de liquide en fonction d'une détection d'un courant d'inspiration par le débitmètre.

5. Nébuliseur médical suivant l'une quelconque des revendications précédentes,
**caractérisé en ce que** les moyens de pompage comprennent une pompe (21) à seringue.

6. Nébuliseur médical suivant la revendication 5,
**caractérisé en ce que** la pompe à seringue est muni d'un réservoir (26) pouvant être couplé de manière amovible à l'extrémité proximale de la buse capillaire.

7. Nébuliseur médical suivant l'une quelconque des revendications précédentes,
**caractérisé en ce que** la buse capillaire peut être reliée à un trajet (5, 8, 9) de courant de gaz d'inspiration d'un appareil (1) mécanique d'aide à la respiration.

8. Nébuliseur médical suivant l'une quelconque des revendications précédentes,
**caractérisé en ce qu'**il est prévu un certain nombre d'autres buses (36, 37) pouvant vibrer individuellement ou collectivement pour stimuler une formation de gouttelettes.
